(19) Europäisches Patentamt
European Patent Office
Office européen des brevets

(11) EP 0 523 543 B1

(12) **EUROPÄISCHE PATENTSCHRIFT**

(45) Veröffentlichungstag und Bekanntmachung des Hinweises auf die Patenterteilung:
**07.02.1996 Patentblatt 1996/06**

(51) Int Cl.[6]: **C09K 19/58**, C09K 19/42, C07D 213/74, C07D 239/42, C07D 295/155

(21) Anmeldenummer: **92111654.7**

(22) Anmeldetag: **09.07.1992**

(54) **Chirale Piperazin-Derivative**

Chiral piperazine derivatives

Dérivés de pipérazines chirales

(84) Benannte Vertragsstaaten:
**CH DE FR GB IT LI NL**

(30) Priorität: **19.07.1991 CH 2164/91**

(43) Veröffentlichungstag der Anmeldung:
**20.01.1993 Patentblatt 1993/03**

(73) Patentinhaber: **F. HOFFMANN-LA ROCHE AG**
**CH-4002 Basel (CH)**

(72) Erfinder: **Stephen, Kelly**
**CH-4313 Möhlin (CH)**

(74) Vertreter: **Cottong, Norbert A. et al**
**CH-4002 Basel (CH)**

(56) Entgegenhaltungen:
**EP-A- 0 213 841**
**EP-A- 0 244 115**
**EP-A- 0 339 414**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 523 543 B1

**Beschreibung**

Die vorliegende Erfindung betrifft neue chirale Dotierstoffe für Flüssigkristalle und deren Herstellung, sowie flüssigkristalline Gemische, die solche Dotierstoffe enthalten und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise können Flüssigkristallphasen für Anzeigevorrichtungen mit verdrillt nematischer Struktur mit optisch aktivem Zusatzstoff dotiert werden, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen auf der Basis von chiral getilteten smektischen Phasen können vorzugsweise aus einem Material mit getilteter smektischer Phase und einem oder mehreren optisch aktiven Dotierstoffen bestehen.

Die elektro-optischen Kennlinien von Flüssigkristall-Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von einem chiralen Dotierstoff, welcher eine mit steigender Temperatur abnehmende Ganghöhe induziert. Eine solche inverse Temperaturabhängigkeit wurde bisher nur für wenige Verbindungen gefunden. Sie kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (US 4 264 148). Allerdings erfordert dies meist einen relativ hohen Anteil an chiralen Dotierstoffen.

Cholesterische Flüssigkristalle reflektieren Licht im wesentlichen nur in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner werden cholesterische Flüssigkristalle auch verschiedentlich für thermochrome Anwendungen gebraucht.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen. Sie sollten mit dem Mesophasetyp des Flüssigkristallmaterials gut kompatibel sein und den Mesophasenbereich nicht zu stark einschränken.

Dotierstoffe für obige Zwecke werden nun gemäss der vorliegenden Erfindung zur Verfügung gestellt.

Die Erfindung betrifft chirale Verbindungen der allgemeinen Formel

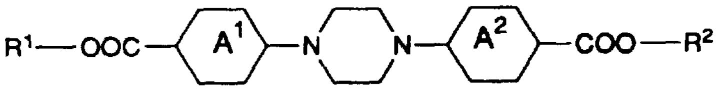

I

worin die Ringe $A^1$ und $A^2$ unabhängig voneinander Pyridin-2,5-diyl, Pyrimidin-2,5-diyl, unsubstituiertes oder einfach oder mehrfach mit Halogen, Cyano und/oder Methyl substituiertes 1,4-Phenylen bedeuten; $R^1$ und $R^2$ unabhängig voneinander Alkyl mit 1 bis 18 Kohlenstoffatomen, in welchem gegebenenfalls 1 oder 2 nicht benachbarte $-CH_2$-Gruppen durch -O-, -COO- und/oder -OOC- und/oder eine $-CH_2-CH_2$-Gruppe durch -CH=CH- ersetzt sein können, darstellen; mit der Massgabe, dass mindestens eine der Gruppen $R^1$ oder $R^2$ chiral sein muss.

Die Verbindungen der Formel I sind leicht in an sich bekannter Weise herstellbar. Sie sind relativ niederviskos und stabil gegen elektrische und magnetische Felder.

Sie sind gut löslich in üblichen Flüssigkristallmaterialien und ermöglichen eine hohe Verdrillung der Flüssigkristallstruktur. Die Klärpunkte von Flüssigkristallen werden bei Zusatz von Verbindungen der Formel I nicht oder nur unwesentlich gesenkt.

Die Verbindungen der Formel I können als Dotierstoffe sowohl in nematischen oder cholesterischen Flüssigkristallen als auch in smektischen Flüssigkristallen angewendet werden.

Der Ausdruck "unsubstituiertes oder einfach oder mehrfach mit Halogen, Cyano und/oder Methyl substituiertes 1,4-Phenylen" umfasst im Rahmen der vorliegenden Erfindung Gruppen wie 1,4-Phenylen, 2-Fluor-1,4-phenylen, 2,3-Difluor-1,4-phenylen, 2-Chlor-1,4-phenylen, 2-Brom-1,4-phenylen, 2-Cyano-1,4-phenylen, 2,3-Dicyano- 1,4-phenylen, 2-Methyl-1,4-phenylen und dergleichen. Bevorzugte Gruppen sind 1,4-Phenylen, 2-Fluor-1,4-phenylen, 2,3-Difluor-1,4-phenylen.

Der Ausdruck "Halogen" bedeutet im Rahmen der vorliegenden Erfindung Fluor, Chlor, Brom oder Jod, insbesondere Fluor und Chlor.

Der Ausdruck "Alkyl mit 1 bis 18 Kohlenstoffatomen, in welchem gegebenenfalls 1 oder 2 nicht benachbarte $-CH_2$-Gruppen durch -O-, -COO-und/oder -OOC- und/oder eine $-CH_2-CH_2$-Gruppe durch -CH=CH- ersetzt sein können" umfasst im Rahmen der vorliegenden Erfindung sowohl geradkettige als auch verzweigte Reste mit 1 bis 18 vorzugsweise 1 bis 12, bzw. 4 bis 12 Kohlenstoffatomen, wie $C_1$-$C_{12}$ Alkyl, $C_4$-$C_{12}$ 2-Methylalkyl, $C_4$-$C_{12}$ 2-(3-Alkenyl), $C_4$-$C_{12}$ 1-(Alkoxycarbonyl)äthyl, $C_5$-$C_{12}$ 1-[(Alkenyloxy)carbonyl]äthyl und dergleichen. Solche Reste sind beispielsweise 2-Butyl, 2-Pentyl, 2-Hexyl, 2-Heptyl, 2-Octyl, 2-Nonyl, 2-Decyl, 2-Undecyl, 2-Dodecyl, 2-Methylbutyl, 2-Methylpentyl, 2-Methylhexyl, 2-Methylheptyl, 2-Methyloctyl, 2-Methylnonyl, 2-Methyldecyl, 2-Methylundecyl, 2-Methyldodecyl, 2-(3-Butenyl), 2-(3E-Pentenyl), 2-(3E-Hexenyl), 2-(3E-Heptenyl), 2-(3E-Octenyl), 2-(3E-Nonenyl), 2-(3E-Decenyl), 2-(3E-Undecenyl), 2-(3E-Dodecenyl), 1-(Methoxycarbonyl)äthyl, 1-(Aethoxycarbonyl)äthyl, 1-(Propyloxycarbonyl)äthyl, 1-(Butoxycarbonyl)äthyl, 1-(Pentyloxycarbonyl)äthyl, 1-(Hexyloxycar-bonyl)äthyl, 1-(Heptyloxycarbonyl)äthyl, 1(Octyloxycarbonyl)äthyl, 1-(Nonyl-oxycarbonyl)äthyl, 1-(Decyloxycarbonyl)äthyl, 1-(Undecyloxycarbonyl)äthyl, 1-(Dodecyloxycarbonyl)äthyl, 1-([trans-2-Propenyloxy]carbonyl)äthyl, 1-([trans-2-Butenyloxy]carbonyl)äthyl, 1-([trans-2-Pentenyloxy]carbonyl)-äthyl, 1-([trans-2-Hexenyloxy]carbonyl)äthyl, 1-([trans-2-Heptenyl-oxy]carbonyl)äthyl, 1-([trans-2-Octenyloxy]carbonyl)äthyl, 1-([trans-2-Nonenyloxy]carbonyl)äthyl, 1-([trans-2-Decenyloxy]carbonyl)äthyl, 1-([trans-2-Undecenyloxy] carbonyl)äthyl, 1-([trans-2-Dodecenyloxy]carbonyl)äthyl und dergleichen.

Bevorzugt werden Verbindungen der Formel I, worin die beiden Ringe $A^1$ und $A^2$ sowie die beiden Reste $R^1$ und $R^2$ jeweils dieselbe Bedeutung haben. Besonders bevorzugt sind Verbindungen der Formel I, worin die Ringe $A^1$ und $A^2$ Pyridin-2,5-diyl, unsubstituiertes oder einfach oder mehrfach mit Halogen, Cyano und/oder Methyl substituiertes 1,4-Phenylen bedeutet.

Weitere besonders bevorzugte Verbindungen der Formel I sind Verbindungen, worin die chiralen Alkylgruppen 4 bis 12, insbesondere 4 bis 8 Kohlenstoffatome aufweisen und worin beide chiralen Alkylgruppen die R-Konfiguration oder beide chiralen Alkylgruppen die S-Konfiguration aufweisen. Vorzugsweise befindet sich das Chiralitätszentrum möglichst nahe am zentralen Ringteil, wodurch ein besonders hohes Verdrillungsvermögen erzielt wird.

Ganz besonders bevorzugt sind Verbindungen der Formel I, worin die Ringe $A^1$ und $A^2$ Pyridin-2,5-diyl bedeuten. Solche Verbindungen zeichnen sich durch gute Löslichkeit und hohe Polarisierbarkeit aus, wodurch die Verbindungen - zusätzlich zu ihrer Anwendbarkeit als Dotierstoffe in nematischen Mischungen - auch für die Anwendung in ferroelektrischen Flüssigkristallen hervorragend geeignet sind.

Weitere bevorzugte Verbindungen der Formel I sind Verbindungen, worin die Ringe $A^1$ und $A^2$ eine unsubstituierte oder eine einfach oder mehrfach mit Halogen, Cyano und/oder Methyl substituierte 1,4-Phenylen-Gruppe bedeutet.

Wie weiter oben erwähnt, sind die Verbindungen der allgemeinen Formel I sehr leicht synthetisch zugänglich. Die Verbindungen der Formel I, worin die Ringe $A^1$ und $A^2$ 1,4-Phenylen bedeuten, können z.B. dadurch hergestellt werden, dass das im Handel erhältliche 1,4-Piperazindiyl-diphenyl in an sich bekannter Weise über Bis-4,4'-(1,4-piperazindiyl) dibenzaldehyd in ein für die Veresterung mit ROH geeignetes Derivat umgesetzt wird (J. Med. Chem. 10, 111 [1967]). Die Verbindungen der Formel I, worin die Ringe $A^1$ und $A^2$ Pyridin-2,5-diyl bedeuten, können durch Kopplung des entsprechenden 6-Chlor-nicotinsäureesters mit Piperazin in einem geeigneten Lösungsmittel hergestellt werden. Die Herstellungsmethoden werden durch die Beispiele weiter veranschaulicht.

Die Erfindung betrifft ebenfalls flüssigkristalline Gemische enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60°C, besonders bevorzugt mindestens etwa 80°C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew.% betragen. Für Anzeigevorrichtungen auf der Basis von Flüssigkristallen mit verdrillt nematischer Struktur ist je nach Zellentyp und -dicke meist eine Ganghöhe von etwa 30-40 µm erforderlich und daher ein entsprechend kleiner Anteil ausreichend. Demgegenüber werden für Anwendungen, welche auf der Reflexion von sichtbarem Licht durch cholesterische Schichten beruhen, Ganghöhen von etwa 0,4-0,6 µm benötigt, was einen entsprechend höheren Anteil an chiralem Dotierstoff erfordert.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien bevorzugt. Grundsätzlich kann aber auch eine einzige flüssigkristalline Verbindung als Trägermaterial verwendet werden, wenn sie eine ausreichend breite Mesophase aufweist.

Als Komponenten für flüssigkristalline Trägermaterialien eignen sich insbesondere Verbindungen der folgenden

allgemeinen Formeln

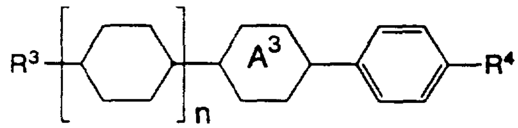

II

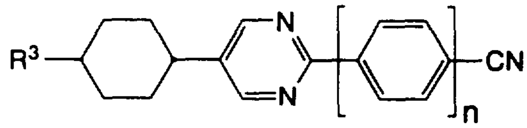

III

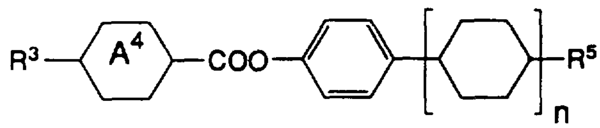

IV

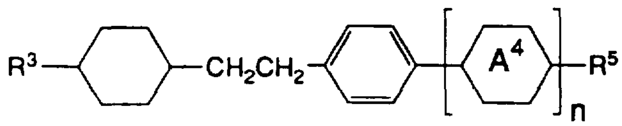

V

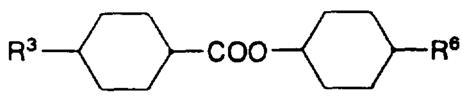

VI

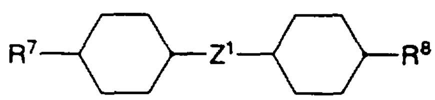

VII

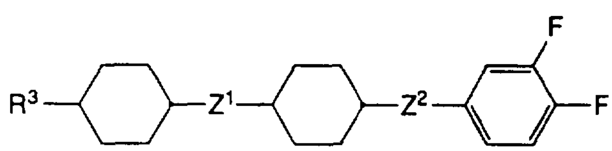

VIII

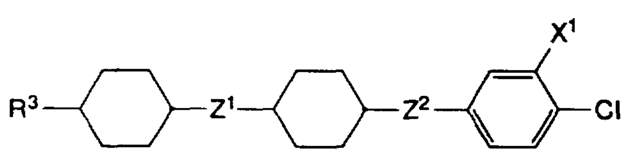

IX

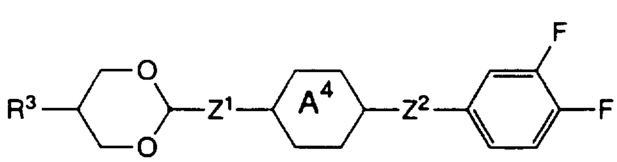

X

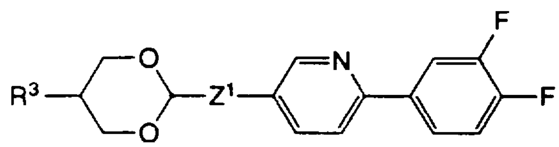

XI

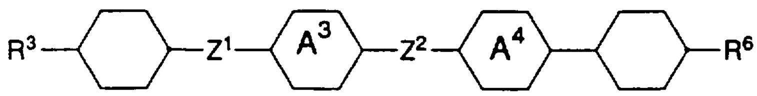

XII

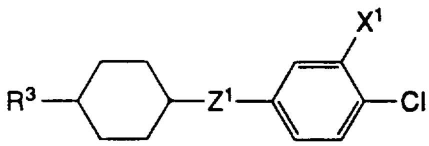

XIII

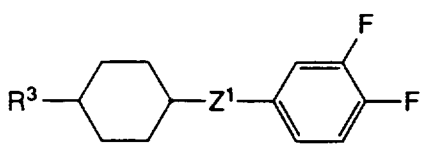

XIV

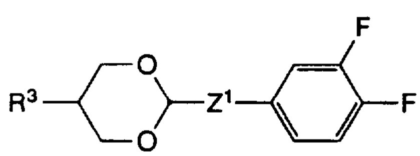

XV

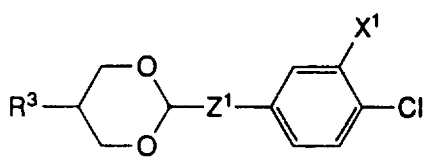

XVI

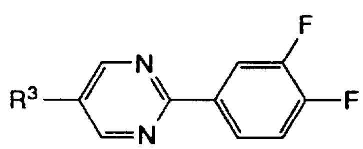

XVII

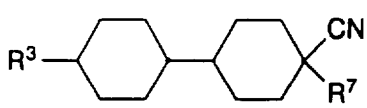

XVIII

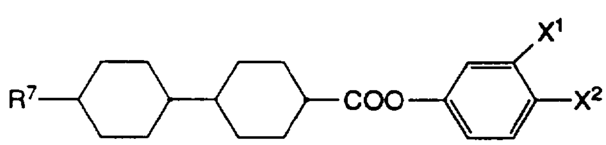

XIX

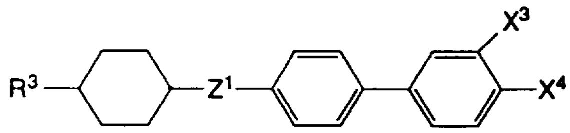

XX

worin Ring $A^3$ 1,4-Phenylen, Pyridin-2,5-diyl, Pyrimidin-2,5-diyl, trans-1,4-Cyclohexylen oder trans-1,3-Dioxan-2,5-diyl bedeutet; Ring $A^4$ 1,4-Phenylen oder trans-1,4-Cyclohexylen darstellt; n die Zahl 0 oder 1 bedeutet; $Z^1$ und $Z^2$ unabhängig voneinander eine Einfachbindung oder $-CH_2CH_2-$ bezeichnen, wobei zwei aromatische Ringe immer durch eine Einfachbindung verknüpft sind; $R^3$ und $R^6$ unabhängig voneinander Alkyl, Alkoxyalkyl, 3E-Alkenyl und an gesättigten Ringen auch 1E-A1kenyl bezeichnen; $R^4$ Alkyl, 3E-Alkenyl, 4-Alkenyl, Alkoxy, 2E-Alkenyloxy, 3-Alkenyloxy, 1-Alkinyl, Cyano, Isothiocyanato oder Fluoro bedeutet; $R^5$ Alkyl, 3E-Alkenyl, 4-Alkenyl oder an trans-1,4-Cyclohexylen auch 1E-Alkenyl oder an 1,4-Phenylen auch Cyano, Isothiocyanato, Alkoxy, 2E-Alkenyl oder 3-Alkenyloxy bezeichnet; $R^7$ Alkyl, 1E-Alkenyl, 3E-Alkenyl oder 4-Alkenyl bedeutet; $R^8$ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt; $X^1$ Wasserstoff, Fluor oder Chlor bedeutet; $X^2$ Cyano, Fluor oder Chlor bezeichnet; $X^3$ Wasserstoff oder Fluor bedeutet; und $X^4$ für Chlor oder Fluor steht.

$R^3$, $R^4$, $R^5$, $R^6$, $R^7$ und $R^8$ besitzen vorzugsweise 1 bis 12 Kohlenstoffatome, besonders bevorzugt 1 bis 7 Kohlenstoffatome.

Der obengenannte Ausdruck "aromatische Ringe" bedeutet 1,4-Phenylen, Pyridin-2,5-diyl oder Pyrimidin-2,5-diyl. Der obengenannte Ausdruck "gesättigte Ringe" bedeuten trans-1,4-Cyclohexylen oder trans-1,3-Dioxan-2,5-diyl.

Die Erfindung wird durch die folgenden Beispiele weiter veranschaulicht. Im Zusammenhang mit Flüssigkristallphasen und Phasenübergängen bedeuten C eine kristalline Phase, N eine nematische Phase, $N^*$ eine cholesterische Phase, S eine smektische Phase und I die isotrope Phase. Die Helixganghöhe wird mit p bezeichnet. Optische Antipoden besitzen jeweils "spiegelbildliche Eigenschaften", d.h. gleiche Schmelzpunkte usw., führen aber zu entgegengesetztem Helixdrehsinn und entgegengesetzter Zirkularpolarisation des reflektierten Lichts.

Beispiel 1

Ein Gemisch von 1,4 g (S)-1-(Aethoxycarbonyl)äthyl-6-chlornicotinsäureester, 0,2 g Piperazin und 25 ml Aethanol wurde während 2 Tage auf 80°C erwärmt. Das Reaktionsgemisch wurde mit 500 ml Wasser versetzt und dann dreimal mit je 100 ml Diäthyläther extrahiert. Die vereinigten organischen Phasen wurden zweimal mit je 500 ml konzentrierter Kochsalzlösung gewaschen, über Magnesiumsulfat getrocknet, filtriert und anschliessend eingeengt. Der Rückstand wurde an Kieselgel mit Toluol/Aethylacetat (Vol. 4:1) chromatographiert. Umkristallisation aus Aethanol ergab reinen Bis-[(S)-1-(äthoxycarbonyl)äthyl]-6,6'-(1,4-piperazindiyl)dinicotinsäureester mit Smp. (C-I) 143°C, $[\alpha]_{589}^{20}$ = +68,3°.

Der als Ausgangsmaterial verwendete (S)-1-(Aethoxycarbonyl)äthyl-6-chlornicotinsäureester wurde wie folgt hergestellt:

a) 15 g 6-Chlornicotinsäure wurden mit 25 ml Thionylchlorid in Toluol 24 Stunden auf 80°C erhitzt. Die erhaltene Lösung wurde unter vermindertem Druck eingedampft, der Rückstand mit 20 ml absolutem Toluol versetzt und die Lösung nochmals unter vermindertem Druck eingedampft. Der feste Rückstand wurde in einer Kugelrohr sublimiert. Dies ergab 14,84 g 6-Chlornicotinsäurechlorid mit Smp. 48°C.

b) 2,5 g 6-Chlornicotinsäurechlorid wurden in 50 ml absolutem Toluol aufgenommen und mit einer Lösung von 1,7 g L(-)-Milchsäure-äthylester in 5 ml absolutem Pyridin versetzt. Das Reaktionsgemisch wurde 1 Stunde zum Rückfluss erhitzt, dann abgekühlt, mit verdünnter Salzsäure versetzt und dreimal mit je 50 ml Dichlormethan extrahiert. Die vereinigten organischen Phasen wurde zweimal mit je 100 ml Wasser gewaschen, über Magnesiumsulfat getrocknet und eingeengt. Das erhaltene Rohprodukt wurde durch Säulenchromatographie an Kieselgel mit Toluol/Aethylacetat (Vol. 4:1) gereinigt. Dies ergab 2,6 g reinen (97%) (S)-1-(Aethoxycarbonyl)-äthyl 6-chlornicotinsäureester mit $[\alpha]_{589}^{20}$ = +14,4°.

In analoger Weise können folgende Verbindungen hergestellt werden:

Bis-[(S)-1-(Methoxycarbonyl)äthyl]6,6'-(1,4-piperazindiyl)dinicotinsäureester, Smp. 169°, $[\alpha]_{589}^{20}$ = +70,9°C;

Bis-[(S)-1-(Propyloxycarbonyl)äthyl] 6,6'-(1,4-piperazindiyl)dinicotinsäureester, Smp. 160°;
Bis-[(S)-1-(Butoxycarbonyl)äthyl] 6,6'-(1,4-piperazindiyl)dinicotinsäureester, Smp. 125°, $[\alpha]_{589}^{20}$ = +61,6°;

Bis-[(S)-1-([trans-2-Butenyl**oxy**]carbonyl)**äthyl** 6,6'-(1,4-piperazindiyl)dinicotinsäureester;
Bis-[(S)-1-([trans-2-Pentenyl**oxy**]carbonyl)**äthyl** 6,6'-(1,4-piperazindiyl)dinicotinsäureester;
Bis-[(S)-1-([trans-2-Hexenyl**oxy**]carbonyl)**äthyl** 6,6'-(1,4-piperazindiyl)dinicotinsäureester, Smp. 130°C;
Bis-[(S)-2-Pentyl] 6,6'-(1,4-piperazindiyl)dinicotinsäureester, Smp. 119°C, $[\alpha]^{20}_{589}$ = +49,8°;
Bis-[(S)-2-Hexyl] 6,6'-(1,4-piperazindiyl)dinicotinsäureester;
Bis-[(S)-2-Heptyl] 6,6'-(1,4-piperazindiyl)dinicotinsäureester;
Bis-[(S)-2-Octyl] 6,6'-(1,4-piperazindiyl)dinicotinsäureester, Smp. 94°C, $[\alpha]^{20}_{589}$ = +65°;
Bis-[(S)-2-Nonyl] 6,6'-(1,4-piperazindiyl)dinicotinsäureester;
Bis-[(R)-1-(Methoxycarbonyl)äthyl] 6,6'-(1,4-piperazindiyl)dinicotinsäureester, Smp. 169°, $[\alpha]^{20}_{589}$ = -70,9°C;
Bis-[(R)- 1-(Propyloxycarbonyl)äthyl] 6,6'-(1,4-piperazindiyl)dinicotinsäureester, Smp. 160°;
Bis-[(R)-1-(Butoxycarbonyl)äthyl] 6,6'-(1,4-piperazindiyl)dinicotinsäureester, Smp. 125°, $[\alpha]^{20}_{589}$ = -61,6°;
Bis-[(R)-1-([trans-2-Butenyl**oxy**]carbonyl)**äthyl** 6,6'-(1,4-piperazindiyl)-dinicotinsäureester;
Bis-[(R)-1-([trans-2-Pentenyl**oxy**]carbonyl)**äthyl** 6,6'-(1,4-piperazindiyl)-dinicotinsäureester;
Bis-[(R)-1-([trans-2-Hexenyl**oxy**]carbonyl)**äthyl** 6,6'-(1,4-piperazindiyl)-dinicotinsäureester, Smp. 130°C;
Bis-[(R)-2-Pentyl] 6,6'-(1,4-piperazindiyl)dinicotinsäureester, Smp. 119°C, $[\alpha]^{20}_{589}$ = -49,8°;
Bis-[(R)-2-Hexyl]6,6'-(1,4-piperazindiyl)dinicotinsäureester;
Bis-[(R)-2-Heptyl]6,6'-(1,4-piperazindiyl)dinicotinsäureester;
Bis-[(R)-2-Octyl]6,6'-(1,4-piperazindiyl)dinicotinsäureester, Smp. 94°C, $[\alpha]^{20}_{589}$ = -65°;
Bis-[(R)-2-Nonyl]6,6'-(1,4-piperazindiyl)dinicotinsäureester.

Beispiel 2

1,1 g Bis-4,4'-(1,4-piperazindiyl)benzoesäurechlorid, 0,5 L(-)-Milchsäureäthylester und 25 ml Toluol wurden in analoger Weise zu Beispiel 1b) umgesetzt. Dies ergab 0,8 g Bis-[(S)-1-äthoxycarbonyl)äthyl] 4,4'-(1,4-piperazindiyl)dibenzoesäureester.

Das als Ausgangsmaterial verwendete Bis-4,4'-(1,4-piperazindiyl)-dibenzoesäurechlorid wurde wie folgt hergestellt:

a) Eine Lösung von 2 g Bis-4,4'-(1,4-piperazindiyl)dibenzaldehyd in 100 ml Aceton wurde tropfenweise mit 10 ml Jones' Reagenz versetzt. Das Gemisch wurde 1 Stunde bei Raumtemperatur gerührt und dann auf 100 ml Wasser gegossen. Der dabei entstandene Niederschlag wurde abfiltriert, portionenweise mit Wasser gewaschen und am Vakuum getrocknet. Das Rohprodukt wurde aus Aethanol umkristallisiert und ergab 1,2 g reine Bis-4,4'-(1,4-piperazindiyl)dibenzoesäure.

b) 1,2 g Bis-4,4'-(1,4-piperazindiyl)dibenzoesäure, 10 ml Thionylchlorid und 100 ml Toluol wurden in analoger Weise zu Beispiel 1a) umgesetzt. Dies ergab 1,1 g Bis-4,4'-(1,4-piperazindiyl)benzoesäurechlorid.

In analoger Weise können folgende Verbindungen hergestellt werden:

Bis-[(S)-1-(Methoxycarbonyl)äthyl] 4,4'-(1,4-piperazindiyl)dibenzoesäureester;
Bis-[(S)-1-(Propyloxycarbonyl)äthyl]4,4'-(1,4-piperazindiyl)dibenzoesäureester;
Bis-[(S)-1-(Butoxycarbonyl)äthyl] 4,4'-(1,4-piperazindiyl)dibenzoesäureester;
Bis-[(S)-2-Pentyl] 4,4'-(1,4-piperazindiyl)dibenzoesäureester;
Bis-[(S)-2-Hexyl]4,4'-(1,4-piperazindiyl)dibenzoesäureester;
Bis-[(S)-2-Heptyl] 4,4'-(1,4-piperazindiyl)dibenzoesäureester;
Bis-[(S)-2-Octyl]4,4'-(1,4-piperazindiyl)dibenzoesäureester;
Bis-[(S)-2-Nonyl] 4,4'-(1,4-piperazindiyl)dibenzoesäureester.

Beispiel 3

Zur Messung der induzierten Ganghöhe (Pitch) und deren Temperaturabhängigkeit in Flüssigkristallmaterialien wurde die folgende nematische Flüssigkristall-Grundmischung BM-1 verwendet.

| | |
|---|---|
| 5,36 Gew.% | 4'-Aethyl-4-cyanobiphenyl, |
| 3,18" | 4'-Propyl-4-cyanobiphenyl, |
| 6,08" | 4'-Butyl-4-cyanobiphenyl, |

| | |
|---|---|
| 6,53" | 4-(trans-4-Propylcyclohexyl)benzonitril, |
| 14,67" | 4-(trans-4-Pentylcyclohexyl)benzonitril, |
| 5,21" | 4-Aethyl-1-(trans-4-propylcyclohexyl)benzol, |
| 16,54" | 4-Aethoxy-1-[2-(trans-4-propylcyclohexyl)äthyl]benzol, |
| 5,60" | 4"-Pentyl-4-cyano-p-terphenyl, |
| 5,71" | 4'-(trans-4-Pentylcyclohexyl)-4-cyanobiphenyl, |
| 15,95" | 1-[2-(trans-4-Butylcyclohexyl)äthyl]-4-(trans-4-pentylcyclo-hexyl)benzol, |
| 4,74" | 4-[2-(trans-4-Butylcydohexyl)äthyl]-4'-(trans-4-pentylcyclohexyl)biphenyl, |
| 7,59" | 4-[2-(trans-4-Butylcyclohexyl)äthyl]-4'-(trans-4-pentylcyclohexyl)-1,1'-äthylendibenzol, |
| 2,84" | trans-4-[2-(trans-4-Propylcyclohexyl)äthyl]cyclohexancarbonsäure 4-cyanophenylester; |

Smp. <-30°C, Klp. (N-I) 90°C; $\Delta\varepsilon$ = 8,5, An = 0,139 und $\eta$ = 22 mPa•s gemessen bei 22°C.
Flüssigkristall-Grundmischung BM-1 wurde jeweils mit einem der folgenden optisch aktiven Dotierstoffe versetzt:

D-1 = Bis-[(R)-2-Octyl] 6,6'-(1,4-piperazindiyl)dinicotinsäureester.

D-2 = Bis-[(S)-1-(äthoxycarbonyl)äthyl] 6,6'-(1,4-piperazindiyl)dinicotinsäureester.

Für die chiral dotierten Gemische wurden die in Tabelle 1 zusammengestellten Ergebnisse erhalten, wobei A, B und C die Parameter der Reihenentwicklung

$$\frac{1}{pc} = A + BT_1 + CT_1^2$$

bezeichnen und p, c und $T_1$ die folgenden Bedeutungen haben:

$T_1$ = T - 22°C

T = Temperatur in °C

p = Ganghöhe in $\mu$m (ein positiver Wert bedeutet rechtsdrehende Helixstruktur und ein negativer Wert bedeutet linksdrehende Helixstruktur).

c = Konzentration des optisch aktiven Dotierstoffes in Gew.-%.

**Tabelle 1**

| Mischung | Dotierstoff | A $[10^{-2} \cdot \mu m^{-1} \cdot Gew.\%^{-1}]$ | B $[10^{-4} \cdot \mu m^{-1} \cdot Gew.\%^{-1} \cdot {}^{\circ}C^{-1}]$ | C $[10^{-6} \cdot \mu m^{-1} \cdot Gew.\%^{-1} \cdot {}^{\circ}C^{-2}]$ | p•c (bei 22°C) $[\mu m \cdot Gew.\%]$ |
|---|---|---|---|---|---|
| M-1 | 7 Gew.% D-1 | 18,43 | -3,94 | -5,55 | +5,4 |
| M-2 | 7 Gew.% D-2 | -21,20 | 5,52 | -2,44 | -4,7 |

Beispiel 5

Zur Messung der spontanen Polarisierbarkeit (Ps) von ferroelektrischen smektischen Flüssigkristallmaterialien wurde die folgende Flüssigkristall-Grundmischung BM-2 verwendet.

| | |
|---|---|
| 4,6 Gew.% | 2-(4-Octyloxyphenyl)-5-heptylpyrimidin |
| 15,7" | 2-(4-Nonyloxyphenyl)-5-nonylpyrimidin |
| 15,7 " | 2-(4-Hexyloxyphenyl)-5-nonylpyrimidin |
| 13,6" | 2-(4-Decyloxyphenyl)-5-decylpyrimidin |
| 25,1" | 4-Decyloxybenzoesäure 4-[2-(trans-4-pentylcyclohexyl)äthyl]-phenylester |
| 12,6" | 4-Undecyloxybenzoesäure 4-[2-(trans-4-pentylcyclohexyl)äthyl]-phenylester |
| 12,6" | 4-Dodecyloxybenzoesäure 4-[2-(trans-4-pentylcyclohexyl)äthyl]phenylester |

Die Flüssigkristall-Grundmischung BM-2 wurde mit einem optisch aktiven Dotierstoff versetzt:

| | |
|---|---|
| D-3 = | Bis-[(S)-2-Octyl] 6,6'-(1,4-piperazindiyl)dinicotinsäureester |
| 93 Gew.% | BM-2 |
| 7 Gew.% | D-3 |

$$\Delta T\ (S_c{}^*)\ \text{-}4°C,\ Ps\ (T_{sc}{}^*\ \text{-}15°) = 88\ nC/cm^2,\ \tau\ 550\ \mu sec.$$

| | |
|---|---|
| $\Delta T\ (S_c{}^*)$ = | Absenkung der Phasenübergangstemperatur $S_c{}^*$ zu $S_A$, N bzw. I. |
| $Ps\ (T_{sc}{}^*\ \text{-}15°)$ = | spontane Polarisierbarkeit, gemessen 15°C unter der Phasenübergangstemperatur |
| $\tau$ = | Schaltgeschwindigkeit |

**Patentansprüche**

**1.** Optische aktive Verbindungen der allgemeinen Formel

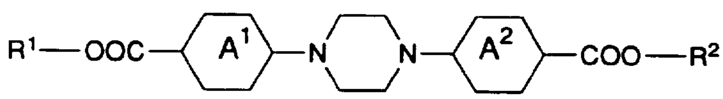

I

worin die Ringe $A^1$ und $A^2$ unabhängig voneinander Pyridin-2,5diyl, Pyrimidin-2,5-diyl, unsubstituiertes oder einfach oder mehrfach mit Halogen, Cyano und/oder Methyl substituiertes 1,4-Phenylen bedeuten; $R^1$ und $R^2$ unabhängig voneinander Alkyl mit 1 bis 18 Kohlenstoffatomen, in welchem gegebenenfalls 1 oder 2 nicht benachbarte $-CH_2$-Gruppen durch -O-, -COO- und/oder -OOC- und/oder eine $-CH_2-CH_2$-Gruppe durch -CH=CH- ersetzt sein können, darstellen; mit der Massgabe, dass mindestens eine der Gruppen $R^1$ oder $R^2$ chiral sein muss.

**2.** Optisch aktive Verbindungen nach Anspruch 1, dadurch gekennzeichnet, dass die Ringe $A^1$ und $A^2$ sowie die Reste $R^1$ und $R^2$ jeweils dieselbe Bedeutung haben.

**3.** Optisch aktive Verbindungen gemäss Anspruch 1 oder 2, dadurch gekennzeichnet, dass die Ringe $A^1$ und $A^2$ Pyridin-2,5-diyl, unsubstituiertes oder einfach oder mehrfach mit Halogen, Cyano und/oder Methyl substituiertes 1,4-Phenylen darstellen.

**4.** Optisch aktive Verbindungen gemäss einem der Ansprüche 1 bis 3, dadurch gekennzeichnet, dass $R^1$ und $R^2$ ein verzweigtes Alkyl mit 4 bis 12 Kohlenstoffatomen, in welchem eine oder zwei nicht benachbarte $-CH_2$-Gruppen durch -O-, -COO- und/oder -OOC- und/oder eine $-CH_2-CH_2$- durch -CH=CH- ersetzt sein können, darstellt.

**5.** Optisch aktive Verbindungen gemäss einem der Ansprüche 1 bis 4, dadurch gekennzeichnet, dass $R^1$ und $R^2$ $C_1$-$C_{12}$ Alkyl, $C_4$-$C_{12}$ 2-Methylalkyl, $C_4$-$C_{12}$ 2-(3-Alkenyl), $C_4$-$C_{12}$ 1-(Alkoxycarbonyl)äthyl oder $C_5$-$C_{12}$ 1-[(Alkenyl-oxy)carbonyl]äthyl darstellen.

6. Optisch aktive Verbindungen gemäss einem der Ansprüche 1 bis 5 dadurch gekennzeichnet, dass die Ringe $A^1$ und $A^2$ Pyridin-2,5-diyl darstellen; und $R^1$ und $R^2$ 2-Alkyl, 1-(Alkoxycarbonyl)äthyl oder 1-[(2-Alkenyloxy)carbonyl]äthyl 4 bis 8 Kohlenstoffatomen bedeuten.

7. Flüssigkristallines Gemisch enthaltend ein flüssigkristallines Trägermaterial und eine oder mehrere optisch aktive Verbindungen der in Anspruch 1 definierten Formel I.

8. Verwendung flüssigkristalliner Gemische gemäss Anspruch 7 für optische und elektro-optische Zwecke.

**Claims**

1. Optically active compounds of the general formula

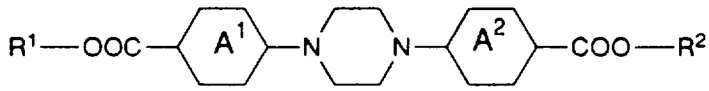

I

wherein rings $A^1$ and $A^2$ each independently signify pyridine-2,5-diyl, pyrimidine-2,5-diyl or 1,4-phenylene, which is unsubstituted or substituted with at least one of halogen, cyano and/or methyl; $R^1$ and $R^2$ each independently represent alkyl with 1 to 18 carbon atoms in which optionally 1 $-CH_2-$ group or 2 non-adjacent $-CH_2-$ groups can be replaced by -O-, -COO- and/or -OOC- and/or one $-CH_2-CH_2-$ group can be replaced by -CH=CH-; with the proviso that at least one of the groups $R^1$ and $R^2$ must be chiral.

2. Optically active compounds according to claim 1, wherein rings $A^1$ and $A^2$ as well as residues $R^1$ and $R^2$ each have the same significance.

3. Optically active compounds according to claim 1 or 2, wherein rings $A^1$ and $A^2$ represent pyridine-2,5-diyl or 1,4-phenylene, which is unsubstituted or substituted with at least one of halgoen, cyano and/or methyl.

4. Optically active compounds according to any one of claims 1 to 3, wherein $R^1$ and $R^2$ represent branched alkyl with 4 to 12 carbon atoms in which one $-CH_2-$ group or two non-adjacent $-CH_2-$ groups can be replaced by -O-, -COO- and/or -OOC- and/or one $-CH_2-CH_2-$ can be replaced by -CH=CH-.

5. Optically active compounds in accordance with any one of claims 1 to 4, wherein $R^1$ and $R^2$ represent $C_1$-$C_{12}$ alkyl, $C_4$-$C_{12}$ 2-methylalkyl, $C_4$-$C_{12}$ 2-(3-alkenyl), $C_4$-$C_{12}$ 1-(alkoxycarbonyl)ethyl or $C_5$-$C_{12}$ 1-[(alkenyloxy)-carbonyl]ethyl.

6. Optically active compounds "according to any one of claims 1 to 5, wherein rings $A^1$ and $A^2$ represent pyridine-2,5-diyl; and $R^1$ and $R^2$ signify 2-alkyl, 1-(alkoxycarbonyl)ethyl or 1. [(2-alkenyloxy)carbonyl]ethyl with 4 to 8 carbon atoms.

7. A liquid crystalline mixture containing a liquid crystalline carrier material and one or more optically active compounds of formula I defined in claim 1.

8. The use of a liquid crystalline mixture in accordance with claim 7 for optical and electro-optical purposes.

**Revendications**

1. Composés optiquement actifs de formule générale

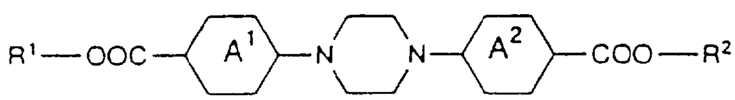

I

dans laquelle les cycles $A^1$ et $A^2$ représentent, indépendamment l'un de l'autre, le radical pyridine-2,5-diyle ou pyrimidine-2,5-diyle, un radical 1,4-phénylène non substitué ou substitué une ou plusieurs fois par un ou des atomes d'halogène et/ou groupes cyano et/ou méthyle; $R^1$ et $R^2$ représentent, indépendamment l'un de l'autre, un radical

alkyle ayant de 1 à 18 atomes de carbone, dans lequel éventuellement 1 ou 2 groupes $-CH_2-$ non contigus peuvent être remplacés par -O-, -COO- et/ou -OOC- et/ou un groupe $-CH_2-CH_2-$peut être remplacé par -CH=CH-; étant entendu qu'au moins l'un des groupes $R^1$ et $R^2$ doit être chiral.

**2.** Composés optiquement actifs selon la revendication 1, caractérisés en ce que les cycles $A^1$ et $A^2$ ainsi que les radicaux $R^1$ et $R^2$ ont, repectivement, la même signification.

**3.** Composés optiquement actifs selon la revendication 1 ou 2, caractérisés en ce que les cycles $A^1$ et $A^2$ représentent le radical pyridine-2,5-diyle ou un radical 1,4-phénylène non substitué ou substitué une ou plusieurs fois par un ou des atomes d'halogène et/ou groupes cyano et/ou méthyle.

**4.** Composés optiquement actifs selon l'une des revendications 1 à 3, caractérisés en ce que $R^1$ et $R^2$ représentent un radical alkyle ramifié ayant de 4 à 12 atomes de carbone, dans lequel un ou deux groupes $-CH_2-$ non contigus peuvent être remplacés par -O-, -COO- et/ou -OOC- et/ou un groupe $-CH_2-CH_2-$ peut être remplacé par -CH=CH-.

**5.** Composés optiquement actifs selon l'une des revendications 1 à 4, caractérisés en ce que $R^1$ et $R^2$ représentent un groupe alkyle en $C_1$-$C_{12}$, 2-méthylalkyle en $C_4$ $C_{12}$, 2-(3-alcényle) en $C_4$ $C_{12}$, 1-(alcoxycarbonyl)éthyle en $C_4$-$C_{12}$ ou 1-[(alcényloxy)carbonyl]éthyle en $C_5$-$C_{12}$.

**6.** Composés optiquement actifs selon l'une des revendications 1 à 5, caractérisés en ce que les cycles $A^1$ et $A^2$ représentent chacun le cycle pyridine-2,5-diyle; et $R^1$ et $R^2$ représentent un groupe 2-alkyle, 1-(alcoxycarbonyl) -éthyle ou 1-[(2-alcényloxy)carbonyl]éthyle ayant de 4 à 8 atomes de carbone.

**7.** Composition de cristaux liquides contenant un matériau de support de type cristal liquide et un ou plusieurs composés optiquement actifs de formule I définie dans la revendication 1.

**8.** Utilisation de compositions de cristaux liquides selon la revendication 7, à des fins optiques et électrooptiques.